# EUROPEAN PATENT SPECIFICATION

(11) **EP 3 678 605 B1**
(45) Date of publication and mention of the grant of the patent: **02.11.2022**
(21) Application number: 18769444.3
(22) Date of filing: 07.09.2018
(51) Int. Cl.: A61F 2/66, A61F 2/74

(54) **PROSTHESIS AND ORTHOSIS**
PROTHESE UND ORTHESE
PROTHESE ET ORTHESE

(30) Priority: 08.09.2017 GB 201714510
(43) Date of publication of application: 15.07.2020
(73) Proprietor: Blatchford Products Limited, Basingstoke Hampshire RG22 4AH (GB); Moog Controls Limited, Tewkesbury Gloucestershire GL20 8NA (GB)
(72) Inventor: BHATTI, Jawaad, Basingstoke Hampshire RG21 5QW (GB); ZAHEDI, Mir Saeed, Barnes London SW13 9BH (GB); MOSER, David, Southampton Hampshire SO16 7PN (GB); BROOKS, Ian, Tewkesbury Gloucestershire GL20 8NA (GB); DUKE, Christopher, Bransgore Hampshire BH23 8BW (GB)
(74) Representative: Jaeger, Michael David
(86) International application number: PCT/GB2018/052535
(87) International publication number: WO 2019/048872

(56) References cited:
- WO-A1-2014/007917
- US-A- 6 117 177
- US-A1- 2014 249 652
- US-A1- 2015 320 573

## Description

### Technical Field

The present invention relates to a prosthesis and orthosis. In particular, the present invention relates to lower limb (ankle or knee) prosthesis and orthosis and a housing for a lower limb prosthesis and orthosis.

### Background to the Invention and Prior Art

Lower limb prostheses are used to restore an amputee's ability to walk, by supporting the weight of an amputee, for example during a stance phase of walking or running, or when standing.

One type of lower limb prosthesis is referred to as an ankle prosthesis, since such a prosthesis corresponds to, and may be designed to mimic, the function of a human ankle.

There have been various developments in lower limb prostheses, including use of powered systems and active response systems as elaborated in relation to known systems described below.

Known lower limb prostheses include those with adaptive control systems for controlling knee flexion or ankle flexion during both stance and swing phases of the walking gait cycle. In WO 99/08621, a prosthetic knee joint has a knee flexion control device including hydraulic and pneumatic parts for controlling knee flexion during the stance phase and swing phase of the gait cycle respectively, the control system including sensors for sensing shin bending moment and knee flexion angle, with corresponding electrical signals being fed to a processing circuit for automatically adjusting the hydraulic and pneumatic flexion control elements. Knee flexion is controlled in the stance phase in response to the activity mode of the amputee, i.e., in response to changes between level walking, walking uphill, and walking downhill, and in the swing phase in response to walking speed. Dynamically variable damping of a prosthetic ankle joint is described in, for example, WO 2008/103917 and related US application number 13/150694 filed 1 June 2011 and published as US 2011/0230975. In this example, the ankle joint includes a hydraulic piston and cylinder assembly providing independent variation of damping resistance in dorsi-flexion and plantar-flexion directions in response to, e.g., ground inclination.

PCT Patent Application published as WO 2013/088142, which claims priority from British Patent Application No. 1208410.9, filed 14 May 2012, abandoned British Patent Application No. 1121437.6, filed 13 December 2011 as well as corresponding US Provisional Patent Applications Nos. 61/580,887 and 61/647,016, filed 28 December 2011 and 15 May 2012 respectively, discloses an integrated lower limb prosthesis for a transfemoral amputee which is integrated in the sense that both knee and ankle joints are controlled, each joint being dynamically adjustable by a processor in response to signals received at different levels in the prosthesis in response to, for instance, signals at the level of the foot or ankle and at a higher level, e.g., on a shin member or at the knee.

Such an electronically controlled prosthesis can include "self-teaching" functions whereby, for instance, the processor can be set to a teaching mode in which data is gathered from sensors on the limb when the amputee performs a walking trial and the data is used to generate a range of settings automatically for use in a normal walking mode. A prosthesis having these features is disclosed in WO 2007/110585 and corresponding US Patent Application No. 12/282,541 and published as US 2009/0057996, filed 11 September 2008.

All of the above prostheses are passive in the sense that their respective control systems vary the resistance in the knee joint or ankle joint, as the case may be, to suit the amputee and the particular actions being performed at any given time. Walking is powered entirely by the muscle power of the amputee.

A powered prosthesis is also known however, i.e., a prosthesis in which the amputee's own muscle power is supplemented with power supplied from an energy source within the prosthesis, in particular from a rechargeable battery. Rotation of the knee joint or ankle joint is driven by one or more actuators powered from the battery. Such prostheses require large batteries and frequent recharging. They also tend to be noisy.

PCT Patent Application published as WO 2014/016583 which claims priority from European patent application 1213035.7 filed 23 July 2012 and US patent application 61/675,347, filed 25 July 2012, describes a further improved powered limb, which teaches the ability to store energy using fluid flow in the hydraulic circuit resulting from joint flexion and to deliver energy to the joint via the hydraulic circuit at different parts of the gait cycle. Such a prosthesis is relatively energy efficient and quiet in operation. The preferred energy storage element is a rechargeable battery and, in this case, the flexion control system preferably includes an electrical machine operable, firstly, as a generator to convert the mechanical energy produced by the above-mentioned energy conversion device into electrical energy for charging the battery and, secondly, as a motor to feed stored electrical energy from the battery to the energy conversion device. In this case, the energy input referred to above can be coupled to the battery to charge the battery from the external energy source.

US-A1-2015/320573 describes a lower limb prosthesis system and a method of controlling the prosthesis system. The prosthesis system has a controller, one or more sensors, a prosthetic foot, and a movable ankle joint member coupled to the prosthetic foot. The movable ankle joint member comprises a hydraulic damping system that provides the ankle joint member damping resistance.

WO-A1-2014/007917 describes a powered lower extremity orthotic, including a shank link coupled to an artificial foot, a knee mechanism connected to the shank link and a thigh link, controlled based on signals from various orthotic mounted sensors such that the artificial foot follows a predetermined trajectory defined by at least one Cartesian coordinate.

US-A-6117177 describes an artificial knee joint including a hollow cylinder in which a piston member is disposed movably. The piston divides the cylinder into upper and lower chambers. The swing phase control member has first and second channels and a fulcrumed lever member bridging the first and second channels. The second channel is communicated with the upper and lower chambers. The first end of the lever member extends into the first channel for movement in response to an air pressure in the first channel. The second end of the lever member has a first throttle valve unit for regulating the air flow through the second channel in response to a movement of the lever member. A pump unit is associated operatively with the piston member for pumping air from the ambient to the first channel in response to a movement of the piston member.

Despite the numerous advantages of improved known prostheses, such as those referred to above, in order to achieve the above-mentioned prostheses there has been a trend to more complex prostheses, many of which require numerous interconnected and inter-operating parts.

As such there are various problems with known ankle prostheses. One main problem that results from the prostheses' complexity is that manufacture, assembly and repair of such prostheses can be difficult.

There is therefore a need for further improvements to known prostheses. In particular, there is a need to further improvements to complex prostheses such as those referred to above.

### Summary of Invention

According to a first aspect of the invention there is provided a prosthesis or orthosis housing, comprising:
a cylinder configured to receive a piston of a piston and cylinder assembly;
a pump section configured to receive part of a pump; and
a plurality of passages connecting the cylinder to the pump section, a plurality of the passages being devoid of bends having a radius of curvature of less than half of the passage width or diameter,
wherein the prosthesis housing is a unitary piece.

A housing having these features can be manufactured using an additive manufacturing method which provides benefits for prostheses and orthoses as described below. For example, using additive manufacturing allows a number of functional parts of the prosthesis or orthosis to be formed in a single manufacturing step and to be physically located in close proximity, resulting in a more compact layout. This is terms allows the housing to be smaller and lighter, which provides numerous benefits to the user as is well known in the art.

The housing may be an ankle prosthesis housing and further comprise a foot attachment section configured for attaching the housing to a foot component.

The prosthesis or orthosis housing may further comprise one or more apertures configured to receive one or more respective valves for controlling fluid flow through one or more of the passages.

The valves may be one or more of an adjustable orifice valve and a check valve.

The prosthesis or orthosis housing may further comprise an aperture configured to receive a switch, such as a solenoid, to be fluidly connected to one of more of the passages such that the switch switches the prosthesis housing between first and second modes of operation.

The first mode of operation may be an active mode where operation of the pump drives the piston and the second mode of operation may be a passive mode where movement of the piston within the cylinder pushes the hydraulic fluid predominantly through the adjustable valves rather than driving the pump.

The pump section may be configured to receive two gears of a gear pump and may further comprise means for mounting the remainder of the pump on the housing. Other types of pumps may be partially or fully received within the housing.

Preferably the bends have a radius of curvature of less than a third of the passage width or diameter and preferably less than a quarter of the passage width or diameter. By manufacturing the housing using an additive manufacturing methods the shape of the internal passages can be optimised to remove sharp corners and reduce the amount of drag experienced by the hydraulic fluid as it passes through the passages and helps mitigate energy losses. This makes the pump and motor more efficient and thereby reduces the energy use, allowing for longer battery life and/or smaller batteries to be used in the prosthesis or orthosis.

The prosthesis housing may be composed of a material having a microstructure indicative that it has been made using additive manufacturing. Since additive manufacturing is used to manufacture the housing, including its apertures and internal passages, fewer manufacturing steps are required to make the housing.

According to a second aspect of the invention there is provided a prosthesis or orthosis comprising:
a housing as described above;
a piston and piston rod housed within the cylinder of the housing; and
a pump mounted to the housing.

The prosthesis or orthosis may further comprise one or more adjustable orifice valve, check valve, solenoid and foot component.

According to a further aspect of the invention there is provided a prosthesis or orthosis unitary housing which is manufactured by an additive manufacturing method, as would be indicative from the microstructure of the material from which the housing has been made.

According to a further aspect of the invention there is provided a prosthesis or orthosis unitary housing comprising:
a cylinder configured to receive a piston of a piston and cylinder assembly;
one or more aperture configured to receive one or valve having an adjustable orifice; and
a plurality of passages connecting the cylinder to the pump section.

This skilled person will readily appreciate that features of the invention described in this application are applicable to both lower and upper limb prostheses as well as orthoses.

### Brief Description of the Drawings

The present invention will now be described by way of example only, and with reference to the accompanying drawings in which:
Figure 1 is a front view of a first embodiment ankle prosthesis housing;
Figure 2 is a first side view of the housing of Figure 1;
Figure 3 a back view of the housing of Figure 1;
Figure 4 is a second side view of the housing of Figure 1;
Figure 5 is a top view of the housing of Figure 1;
Figure 6 is a lower view of the housing of Figure 1;
Figure 7 is a first horizontal cross-section corresponding to line A-A in Figure 2;
Figure 8 is a second horizontal cross-section corresponding to line B-B of Figure 4;
Figure 9 is a third horizontal cross-section corresponding to line C-C of Figure 4;
Figure 10 is a vertical cross-section corresponding to line D-D of Figure 3;
Figure 11 is a first side view of an embodiment of a lower limb prosthesis assembly containing the first embodiment ankle prosthesis housing of Figure 1;
Figure 12 is a second side view of the prosthesis assembly of Figure 11;
Figure 13 is a partial cross-section through the prosthesis assembly of Figures 11 and 12;
Figure 14 is a hydraulic circuit corresponding to the prosthesis assembly of Figures 11 and 12;
Figure 15 is a first perspective view of a first embodiment knee orthosis; and
Figure 16 is a second perspective view of the knee orthosis of Figure 15.

### Detailed Description of the Drawings

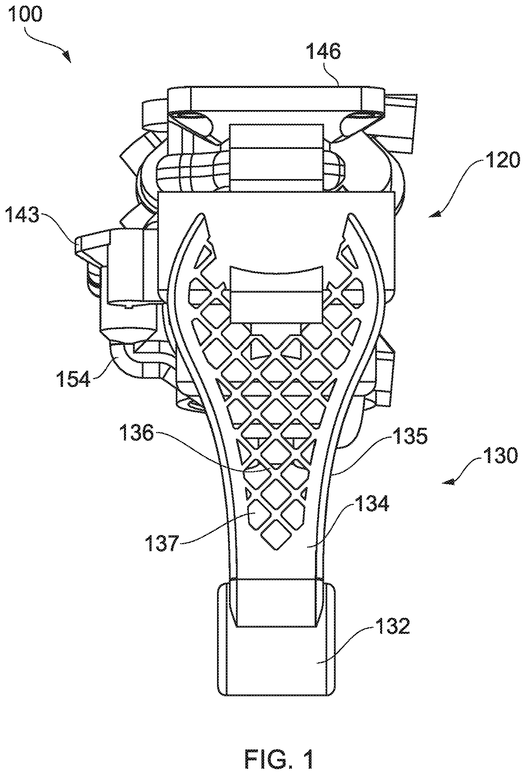

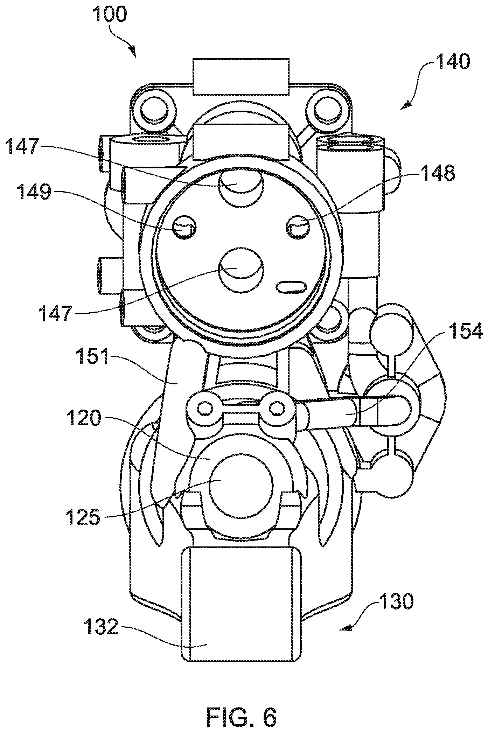

Figures 1 to 6 are respective: front; first side; back; second side; top; and lower views of a first embodiment ankle prosthesis housing 100.

The first embodiment ankle prosthesis housing 100 comprises three main sections: a piston and cylinder assembly (PACA) section 120, an ankle flexion pivot interface section 130, and an accessory interface section 140.

The PACA section 120 comprises an outer wall 122 which defines a first, lower cylinder and a second upper cylinder. The first, lower cylinder has a diameter less than the second, upper cylinder and is configured to receive a piston and piston rod.

The first, lower cylinder comprises a cylindrical wall 122 and a circular base portion 121. An aperture 125 is formed in the base 121 of the first, lower cylinder. The aperture 125 is configured to receive a piston rod which extends from a piston slidably mounted in the first, lower cylinder. A first port 126 is formed in the base 121 of the first, lower cylinder. A second port 127 is formed in an upper portion of the first, lower cylinder.

The second, upper cylinder is configured to receive a cap which closes the top of the first, lower cylinder. The cap includes means for attaching the ankle prosthesis housing 100 to a shin component, such as a pyramid alignment interface or a shin clamp.

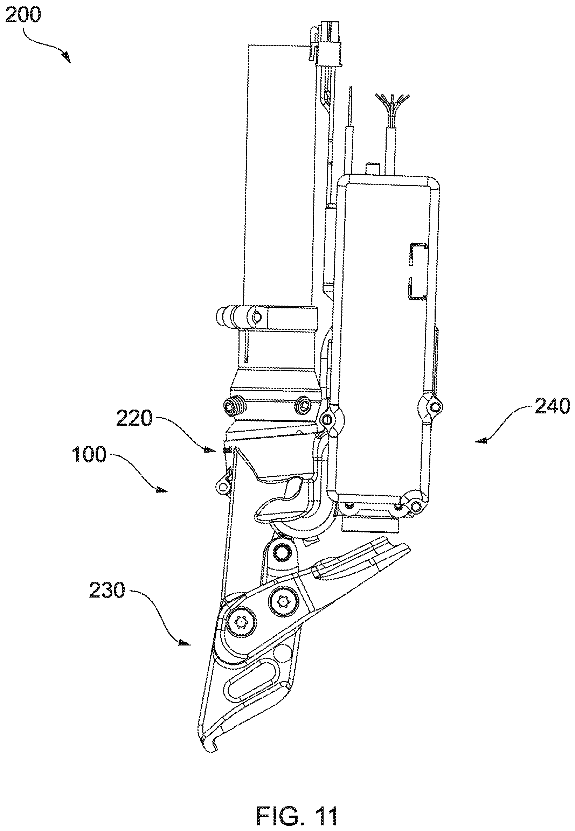

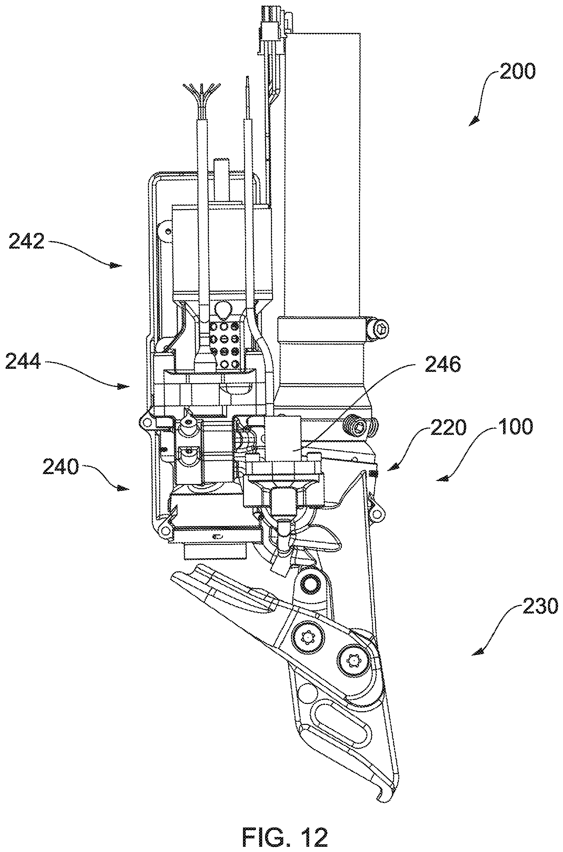

Flexion pivot interface section 130 is configured so that the first embodiment ankle prosthesis housing 100 can be pivotally attached to a foot component 230 (partially shown in Figures 11 and 12). As shown in Figures 1 to 4, the flexion pivot interface section 130 comprises a hollow cylindrical rod 132 and a bridging piece 134. Cylindrical rod 132 is configured to receive a connecting piece attached to the foot component 230, so that the foot component 230 can be attached to the cylindrical rod 132, whilst being at least partially free to rotate about a central axis of cylindrical rod 132. Bridging piece 134 is configured to connect the cylindrical rod to the PACA section 120. As shown in Figure 1, bridging piece 134 comprises an outer rim 135 and a supporting section or plurality of supporting struts 136. The outer rim 135 substantially defines a water-droplet shape as shown in Figure 1. The supporting section or struts 136 is elongate and configured to connect one part of the outer rim 135 to another part of the outer rim 135. The supporting section or struts are configured such that they can translate a force from one side of the outer rim 135 to an opposite side of the outer rim 135. The supporting struts 136 are arranged such that they define an aperture or series of apertures 137. The supporting struts 136 are arranged in a regular arrangement, such as the grid arrangement shown in Figure 1. Although a regular array of apertures has been shown, any suitable arrangement, shape, or size of aperture 135 may be used. This structure of the flexion pivot interface section 130 provides the section 130 with structural strength whilst minimising its weight.

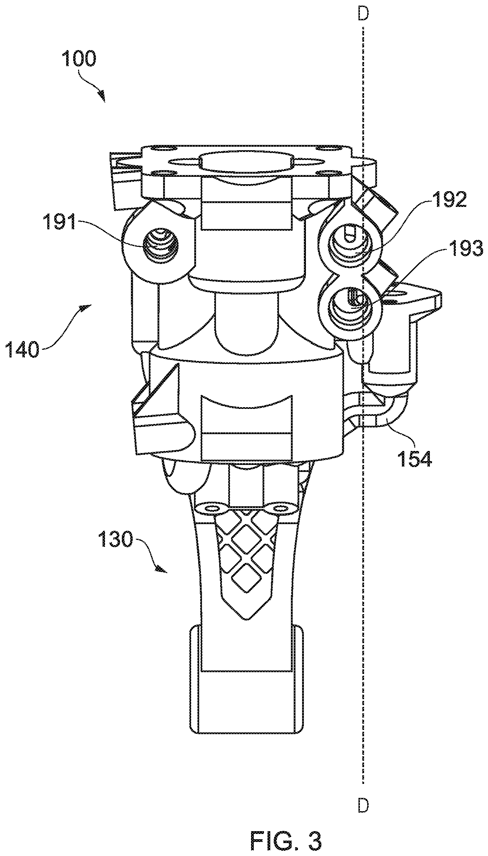

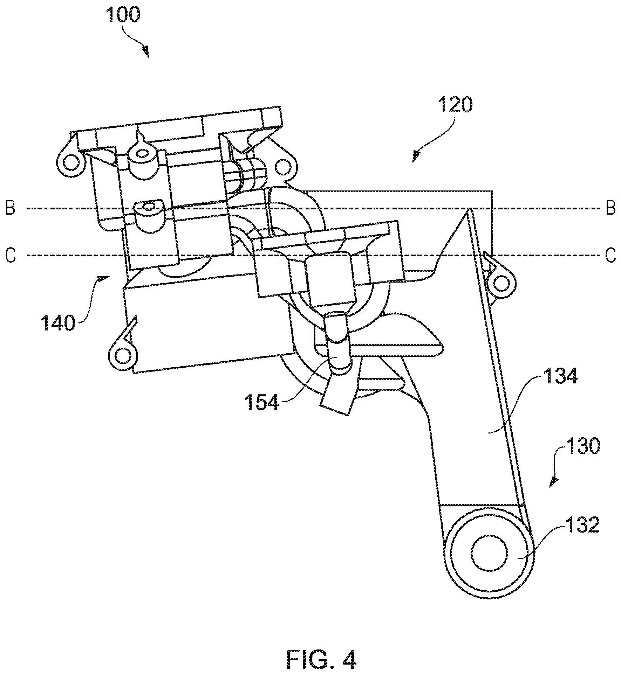

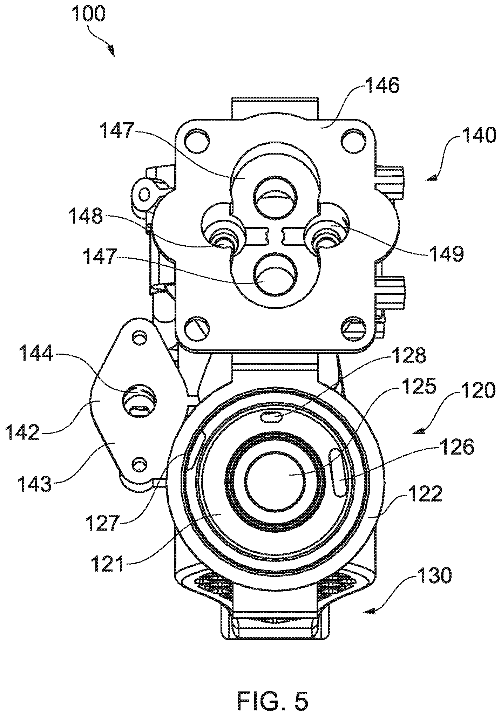

The accessory interface section 140 is configured such that one or more ankle prosthesis housing accessories may be attached thereto. Ankle prosthesis housing accessories may include, but are not limited to: a pump; a gear arrangement; an actuator; a motor; a sensor; an inertial measurement unit (IMU); an electronic component; a valve; and an accumulator. As best seen in Figures 4 and 5, the accessory interface section 140 comprises an actuator interface section 142. The actuator interface section 142 is configured such that a solenoid (shown in Figure 12) can be attached thereto. The actuator interface section 142 comprises a platform section 143. Platform 143 is substantially planar. Platform 143 defines an aperture 144 for receiving part of the solenoid. The accessory interface section 140 also comprises a pump interface section 146. Pump interface section 146 comprises a pump section which is configured to receive a part of a pump, such that the pump interface section 146 and the part of a pump attached thereto together form the pump. The pump interface section 146 defines a cavity, configured to receive a pair of gears which together form a gear pump. As shown in Figure 5, such a cavity defines first and second gear casings 147, each gear casing having a respective aperture configured to receive a gear driver or gear attachment. The accessory interface section 140, as best seen in Figure 3, further includes a first valve opening 191, a second valve opening 192 and a third valve opening 193.

The first embodiment ankle prosthesis housing 100 is configured such that accessories attached to the accessory interface section 140 are fluidly connected to other sections of the first embodiment ankle prosthesis housing 100, through the first embodiment ankle prosthesis housing 100. The term "fluidly connected" as used herein refers to a connection along which fluid, such as hydraulic fluid, can pass. As an example, one end of a hollow tube is fluidly connected to an opposite end of a hollow tube, so that fluid can pass within the tube from one end to the other. The first embodiment ankle prosthesis housing 100 achieves such fluid connections by provision of a series of passages. The first embodiment ankle prosthesis housing 100 is configured or adapted to have one or more curved fluid passages. The first embodiment ankle prosthesis housing 100 is configured or adapted to have one or more fluid passages which are devoid of any sharp bends such as a right angle. The one or more fluid passages may be devoid of any bends having a radius of curvature of less than half of the passage width or diameter. The one or more fluid passages may be devoid of any bends having a radius of curvature of less than a third of the passage width or diameter. The one or more fluid passages may be devoid of any bends having a radius of curvature of less a quarter of the passage width or diameter. The one or more fluid passages may be devoid of any bends having a radius of curvature of less than a tenth of the passage width or diameter.

As shown in Figures 1 to 10, the first embodiment ankle prosthesis housing 100 comprises: a first passage 151, a second passage 152, a third passage 153, and a fourth passage 154.

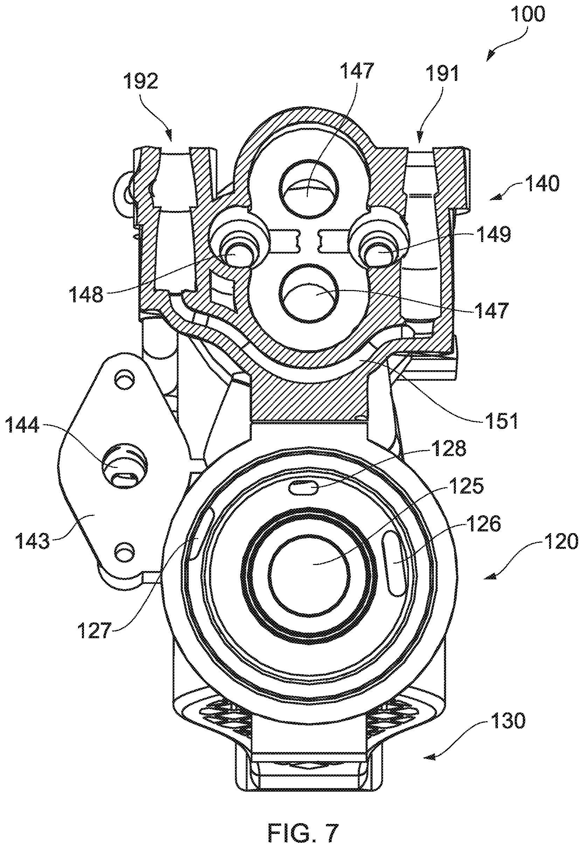

First passage 151 is configured to fluidly connect components attached to the accessory interface section 140 to each other. As best seen in Figure 7, first passage 151 is configured to fluidly connect the first valve opening 191 to second valve opening 192.

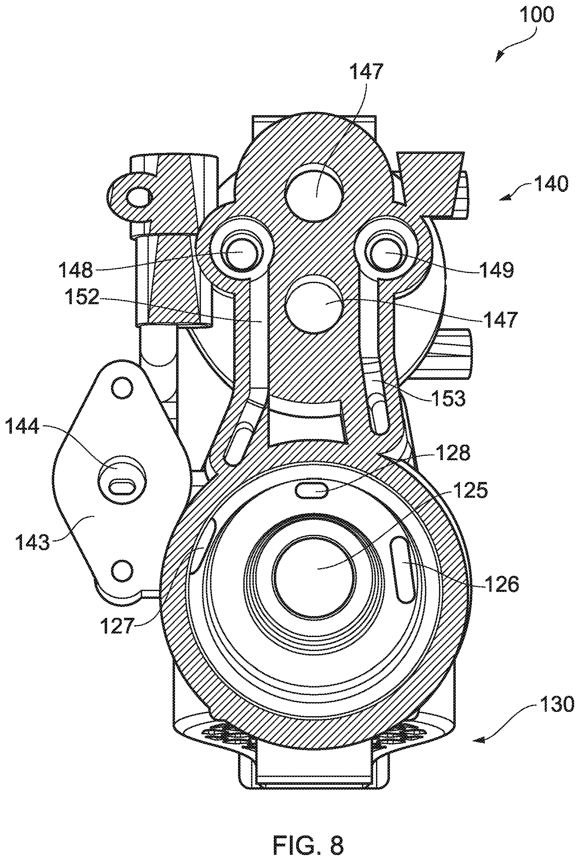

Second passage 152 is configured to fluidly connect PACA section 120 to accessory interface section 140. As best seen in Figure 8, second passage 152 is configured to fluidly connect second port 127 to a third port 148.

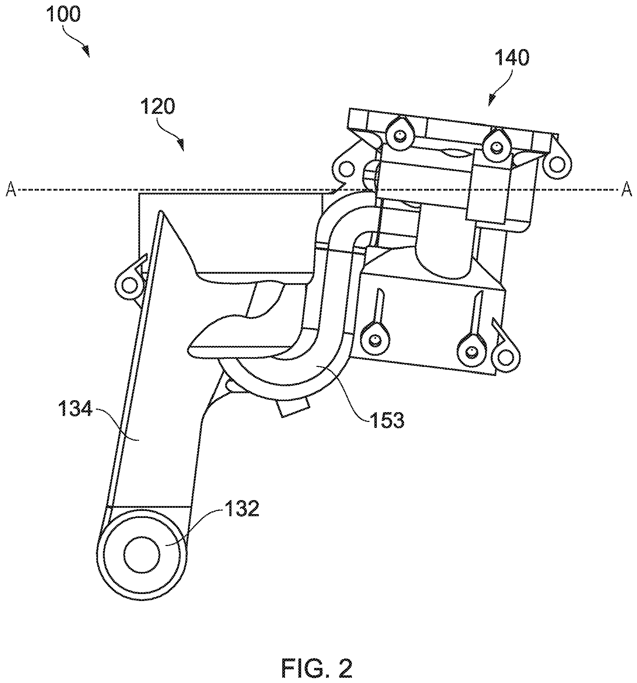

Third passage 153 is configured to fluidly connect PACA section 120 to accessory interface section 140. As best seen in Figures 2, 5, and 8, third passage 153 is configured to fluidly connect first port 126 to a fourth port 149.

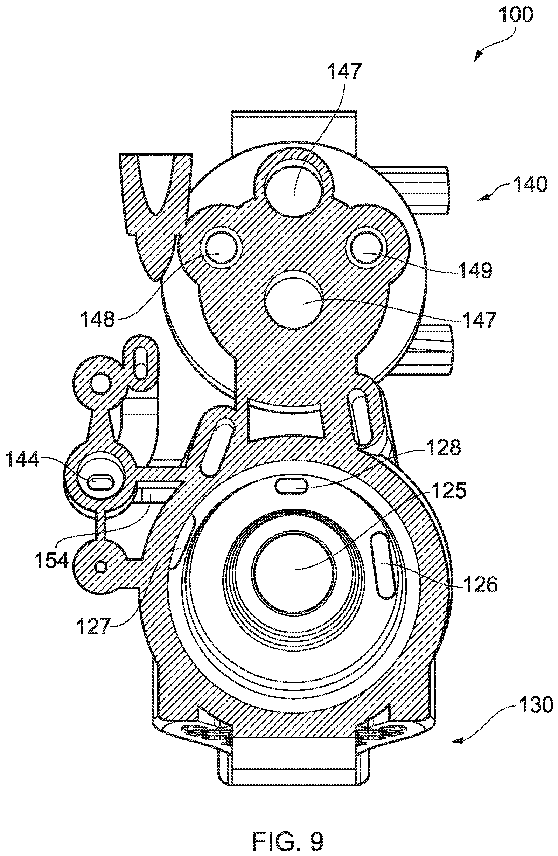

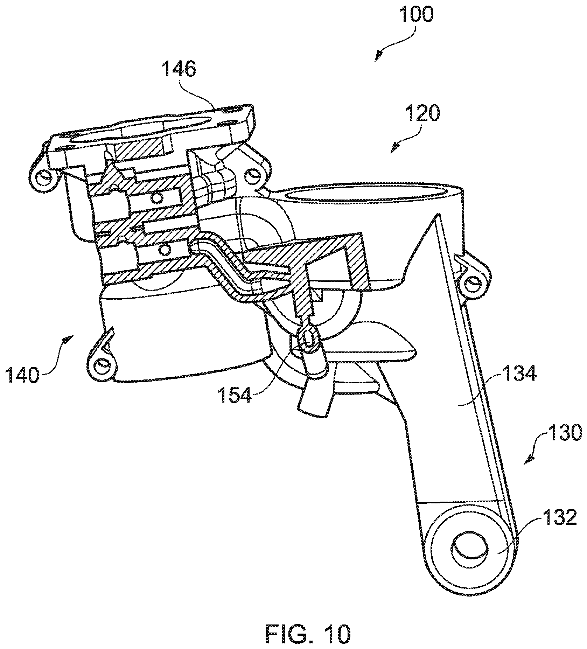

Fourth passage 154 is configured to fluidly connect components attached to the accessory interface section 140 to the PACA section 120. As best seen in Figures 9 and 10, fourth passage 154 is configured to fluidly connect aperture 144 to a sixth port 128.

The first embodiment ankle prosthesis housing 100 is a single unitary piece of metal or alloy, i.e., is formed/manufactured as a single piece/part/manifold without joints. However, as a skilled person will appreciate, the first embodiment ankle prosthesis housing 100 is not limited to these materials and any appropriate material can be used. Suitable metals and alloys include: titanium; aluminum; stainless steels. The first embodiment ankle prosthesis housing 100 is formed using an additive manufacturing technique, such as but not limited to: material jetting, binder jetting, extrusion, and powder bed fusion. Since the housing is made using additive manufacturing its material has a microstructure indicative that it was made by additive manufacturing, i.e., it is apparent from inspection of the housing that the housing has been manufactured using an additive manufacturing method. Furthermore, since the manufacture of the housing by additive manufacturing includes formation of the passages and apertures within the housing as part of the additive manufacturing process, whereas traditionally passages and apertures are drilled into a cast or machined block or manifold. Such a process requires steps to be taken after the manifold is initially formed to create the passages, and these passages, when drilled, will be formed of straight runs which join at harsh angles. Some or all of these straight runs will need to be sealed with plugs, which may leak. In contrast, by forming the housing of the present invention as a unitary manifold/housing there is no need to take additional steps of drilling passages and these passages can include more gentle curves, thereby providing less resistance to fluid flow. Additionally, by using additive manufacturing the physical location and proximity of sections of the housing can be optimised.

The first embodiment ankle prosthesis housing 100 may have been subjected to a form of post-processing strengthening treatment, including but not limited to heat treatment. The first embodiment ankle prosthesis housing 100 may also have been subjected to a form of surface finishing treatment. The skilled person will readily understand that notwithstanding the prosthesis housing described herein is an ankle prosthesis housing, the teachings of this application can be applied to produce a housing of a knee or other prosthesis or orthosis.

As shown in Figures 11 and 12, the first embodiment ankle prosthesis housing 100 is configured for attachment to prosthesis assembly components to form an ankle prosthesis assembly 200. The prosthesis assembly 200 further includes a foot component 230, a piston assembly 220, and one or more accessory components 240.

The one or more accessory components 240 include a motor 242, a pump 244, a solenoid component 246, and adjustable and non-return valves as shown in Figures 11-13.

Motor 242 is attached to pump 244, and configured such that actuation of motor 242 drives the pump 244. As shown in Figure 12, pump 244 is at least partially integrated into the first and second gear casings 147 of the first embodiment ankle prosthesis housing 100.

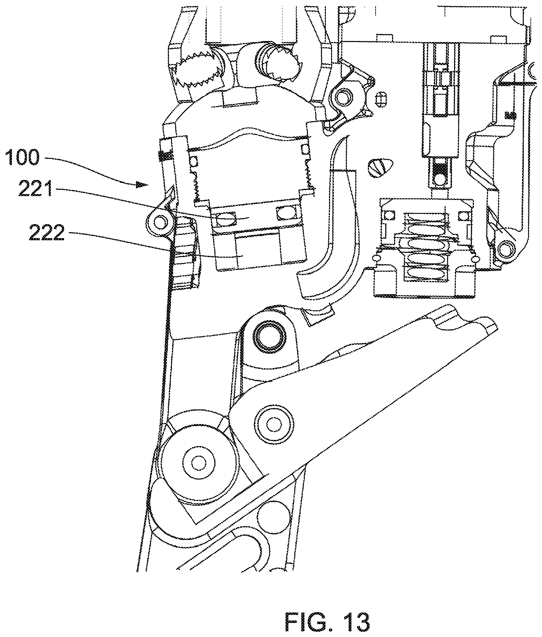

The pump 244 is fluidly connected by means of second passage 152 to piston the assembly 220. Specifically, the pump 244 is fluidly connected by means of second passage 152 to a first side of piston assembly 220. In the present embodiment the first side of the piston assembly 220 is an upper side of the piston 221. The piston assembly 220 is shown in Figure 13, in which the piston rod 222 passes through the aperture 125 in the base 121 of the first, lower cylinder of the PACA section 120.

The pump 244 is fluidly connected by means of third passage 153 to piston assembly 220. Specifically, the pump 244 is fluidly connected by means of third passage 153 to a second side of piston assembly 220. In the present embodiment the second side of the piston assembly 220 is a lower side of the piston 221.

The solenoid component 246 is fluidly connected to the pump 244 and piston assembly 220 by means of the fourth passage 154.

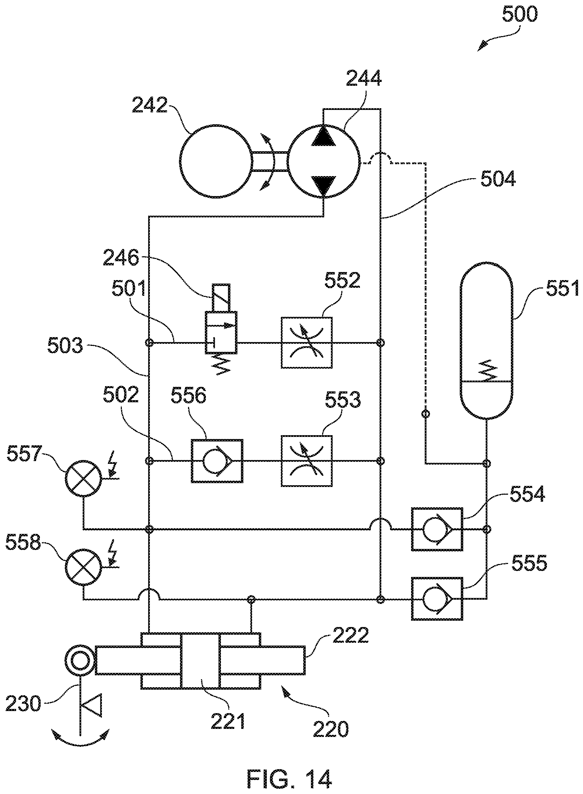

Figure 14 shows a hydraulic circuit 500 corresponding to the prosthesis assembly 200 shown in Figures 11 to 13. The hydraulic circuit 500 comprises a piston assembly 220 having a piston 221 and piston rod 222. The hydraulic circuit 500 also comprises a motor 242, pump 244, and a solenoid component 246.

The hydraulic circuit 500 also comprises accumulator 551, a first adjustable orifice 552, a second adjustable orifice 553, a first check valve 554, a second check valve 555, a third check valve 556, first pressure transducer 557, second pressure transducer 558, and foot component 230. The first adjustable orifice 552 is disposed within the third valve opening 193 and provides variable dorsiflexion resistance. The second adjustable orifice 553 is disposed within the second valve opening 192 and provides variable plantarflexion resistance. The hydraulic circuit also comprises first hydraulic line 501, second hydraulic line 502, third hydraulic line 503, and fourth hydraulic line 504.

First hydraulic line 501 corresponds to fourth passage 154. Third hydraulic line 503 corresponds to first and second passages 151, 152.

First valve opening 191 is configured to receive third check valve 556. Second valve opening 192 is configured to receive second adjustable orifice 553. Third valve opening 193 is configured to receive first adjustable orifice 552.

The prosthesis assembly 200 comprises four main operating modes, two of which are active (i.e. receive an energy input from the motor 242) and two of which are passive (i.e. where the motor 242 is not engaged). Switching between the active and passive modes is achieved by actuating the solenoid 246.

The first mode is passive plantarflexion (PPF) mode. The ankle prosthesis assembly 200 is configured to operate in the first mode following heel strike when the prosthesis assembly is in use. In this mode, the solenoid 246 blocks its hydraulic path, so that fluid cannot flow through the first hydraulic line 501, the solenoid component 246 or through the first adjustable orifice 552. In this mode, the piston 221 and piston rod 220 move within the cylinder upwards (to the right hand side of the schematic shown in Figure 14) under force applied by the amputee to the heel. This causes fluid to flow through the fourth hydraulic line 504, through the third hydraulic line 503, and through the second hydraulic line 502 to the underside (left hand side as shown in Figure 14) of the piston 221. In other words, the hydraulic fluid is pushed by the piston 221 to flow anticlockwise, primarily through the second adjustable orifice 553 (variable plantarflexion resistance valve) and past the third check valve 556, with some hydraulic fluid driving the pump 244. The motor 242 may be configured to prevent this from happening, to prevent the pump 244 from being back-driven.

The second mode is passive dorsiflexion (PDF) mode. In this mode the piston 221 is driven to the left in Figure 14 as the amputee continues the gait cycle with the shin component moving over the foot component 230. In this mode, the solenoid 246 is unblocked, so that fluid can flow through the solenoid component 246, through the first adjustable orifice 552 (variable dorsiflexion resistance valve), and through the first hydraulic line 501. The fluid is prevented from flowing through the second adjustable orifice 553 by the third check valve 556. In this mode, the piston 221 and piston rod 220 are caused to move within the cylinder, downwards (to the left hand side of the schematic shown in Figure 14). This causes fluid to flow through first hydraulic line 501, to the upper (right hand side as shown in figure 14) of the piston 221. In other words, the hydraulic fluid is pushed by the piston 221 to flow clockwise, primarily through the solenoid 246 and first adjustable orifice 552 (variable dorsiflexion resistance valve), with some hydraulic fluid driving the pump 244 through third hydraulic line 503 and through fourth hydraulic line 504. The motor 242 may be configured to prevent the pump from being back-driven.

The third mode is active plantarflexion (APF) mode. The prosthesis assembly 200 is configured to operate in the third mode towards the end of the stance phase, and can be used to rotate the shin component about the foot carrier 230, before toe-off. In this mode, the solenoid 246 is blocked, so that fluid cannot flow through the first hydraulic line 501, through solenoid component 246 or through first adjustable orifice 552. In this mode, motor 242 actuates pump 244, which forces fluid through third hydraulic line 503, and causes the piston 221 and piston rod 220 to move within the cylinder, upwards (to the right hand side of the schematic shown in Figure 14). This causes fluid to flow through fourth hydraulic line 504, from the upper (right hand side as shown in Figure 14) of the piston 221.

The fourth mode is active dorsiflexion (ADF) mode. This occurs during the swing phase to lift the toe as the foot swings forward. In this mode, the solenoid component 246 is blocked, so that fluid cannot flow through the first hydraulic line 501, through solenoid component 246 or through first adjustable orifice 552. In this mode, motor 242 actuates pump 244 in the opposite direction to which it is driven in the third mode, which forces fluid through fourth hydraulic line 504, and causes the piston 221 and piston rod 220 to move within the cylinder, downwards (to the left hand side of the schematic shown in Figure 14). This causes fluid to flow from the lower (left hand side as shown in figure 14) of the piston 221, through third hydraulic line 503 back to the pump 244. Some fluid may flow through second hydraulic line 502 and the second adjustable orifice 553 in this mode.

Due to the arrangement shown by the hydraulic circuit in Figure 14, there are losses when ADF occurs by bypass flow through second hydraulic line 502, through second adjustable orifice 553 and third check valve 556, but not when APF occurs.

Although a dry prosthesis assembly 200 has been shown in Figures 11 and 12, it should be understood that features of the prosthesis assembly 200 could be configured to provide a wet system, i.e., one in which movable components are surrounded by an outer housing, in which oil or a suitable fluid is held.

Although a specific form and arrangement of ankle prosthesis and ankle prosthesis housing is shown in the Figures, it will be appreciated that various aesthetic, structural, dimensional and spatial changes could be made to the device shown whilst still performing the function of the present invention as defined in the appended claims.

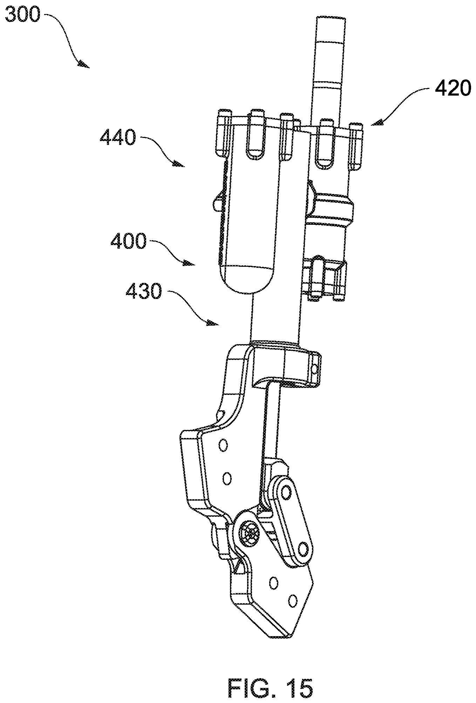

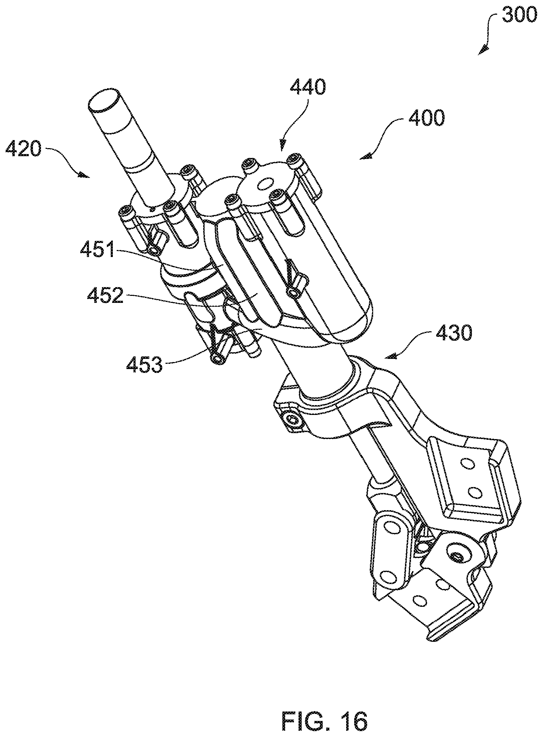

The principles governing the arrangement of the ankle prosthesis housing described above can also be applied to other prostheses and orthoses. One example of such an orthosis is a knee orthosis 300, shown in Figures 15 and 16.

The orthosis shown in Figures 15 and 16 comprises a housing 400. Housing 400 comprises an upper connection section 420, a lower connection section 430, and an accessory interface 440.

Similar to the first embodiment ankle prosthesis housing 100 described above, sections of the housing 400 are fluidly connected to each other by means of fluid passages. Similar to the first embodiment ankle prosthesis housing 100 described above, the housing 400 is configured or adapted to have one or more curved fluid passages. The knee orthosis housing 400 is configured or adapted to have one or more fluid passages which are devoid of any sharp bends. The one or more fluid passages may be devoid of any bends having a radius of curvature of less than half of the passage width or diameter. The one or more fluid passages may be devoid of any bends having a radius of curvature of less than a third of the passage width or diameter. The one or more fluid passages may be devoid of any bends having a radius of curvature of less a quarter of the passage width or diameter. The one or more fluid passages may be devoid of any bends having a radius of curvature of less than a tenth of the passage width or diameter. As shown in Figure 16, there may be a first knee passage 451, a second knee passage 452, and a third knee passage 453.

Similar to the first embodiment ankle prosthesis housing 100 described above, the knee orthosis housing 400 may be a single unitary piece of metal or alloy. The knee orthosis housing 400 is not limited to these materials and any appropriate material can be used. Suitable metals and alloys include: titanium, aluminium, stainless steel. The knee orthosis housing 400 may be formed using an additive manufacturing technique, such as but not limited to: material jetting, binder jetting, extrusion, and powder bed fusion. The knee orthosis housing 400 may have been subjected to a form of post-processing strengthening treatment, including but not limited to heat treatment. The knee orthosis housing 400 may also have been subjected to a form of surface finishing treatment.

Although a specific form and arrangement of ankle prosthesis and knee orthosis housing is shown in the Figures, it will be appreciated that various aesthetic, structural, dimensional and spatial changes could be made to the device shown whilst still performing the function of the present invention as defined in the appended claims.

## Claims

1. A prosthesis (100) or orthosis (400) housing, comprising:
a cylinder configured to receive a piston (221) of a piston and cylinder assembly (120, 220);
a pump section configured to receive part of a pump (244); and
a plurality of passages (151, 152, 153, 154, 451, 452, 453) connecting the cylinder to the pump section,
**characterised in that** a plurality of the passages (151, 152, 153, 154, 451, 452, 453) are devoid of bends having a radius of curvature of less than half of the passage width or diameter, and
wherein the prosthesis (100) or orthosis (400) housing is a unitary piece.

2. A prosthesis housing as claimed in claim 1, wherein the prosthesis housing (100) is an ankle prosthesis housing and further comprises a foot attachment section (132) configured for attaching the housing to a foot component (230).

3. A prosthesis housing as claimed in claim 1 or 2 or an orthosis housing as claimed in claim 1, further comprising one or more apertures (191, 192, 193) configured to receive one or more respective valves for controlling fluid flow through one or more of the passages.

4. A prosthesis or orthosis housing as claimed in claim 3, further comprising one or more adjustable orifice valves (552, 553).

5. A prosthesis or orthosis housing as claimed in claim 3 or 4, further comprising one or more check valves (554, 555, 556).

6. A prosthesis or orthosis housing as claimed in any one of the preceding claims, further comprising an aperture configured to receive a switch (246) to be fluidly connected to one of more of the passages (501) such that the switch switches the prosthesis housing between first and second modes of operation.

7. A prosthesis or orthosis housing as claimed in claim 6, further comprising a solenoid (246) as the switch in the aperture.

8. A prosthesis or orthosis housing as claimed in any of the preceding claim, wherein the pump section is configured to receive two gears of a gear pump and further comprises means for mounting the remainder of the pump on the housing.

9. A prosthesis or orthosis housing as claimed in any one of the preceding claims, wherein a plurality of the passages (151, 152, 153, 154, 451, 452, 453) are devoid of any bends having a radius of curvature of less than a third of the passage width or diameter and preferably less than a quarter of the passage width or diameter.

10. A prosthesis or orthosis housing according to any one of the preceding claims, wherein the housing is composed of a material having a microstructure indicative that it has been made using additive manufacturing.

11. A prosthesis or orthosis comprising:
a housing as claimed in any one of the preceding claims;
a piston (221) and piston rod (220) housed within the cylinder of the housing; and
a pump (244) mounted to the housing.

12. A prosthesis or orthosis as claimed in claim 11 when dependent on claim 3, further comprising one or more adjustable orifice valve (552, 553).

13. A prosthesis or orthosis as claimed in claim 11 or 12 when further dependent on claim 4, further comprising one or more check valve (554, 555, 556.

14. A prosthesis or orthosis as claimed in claim 11, 12 or 13 when further dependent on claim 7, further comprising a solenoid (246).

15. A prosthesis as claimed in any one of claims 11 to 14 when further dependent on claim 2, further comprising a foot component (230).

## Patentansprüche

1. Gehäuse für eine Prothese (100) oder eine Orthese (400), das folgendes umfasst:
einen Zylinder, der konfiguriert ist, um einen Kolben (221) einer Kolben- und
Zylinderanordnung (120, 220) aufzunehmen;
einen Pumpenabschnitt, der konfiguriert ist, um einen Teil einer Pumpe (244) aufzunehmen; und
eine Vielzahl von Kanälen (151, 152, 153, 154, 451, 452, 453), über die der Zylinder mit dem Pumpenabschnitt verbunden ist, **dadurch gekennzeichnet, dass** eine Vielzahl der Kanäle (151, 152, 153, 154, 451, 452, 453) keine Krümmungen mit einem Krümmungsradius von weniger als der Hälfte der Kanalbreite oder des Kanaldurchmessers aufweist, und
wobei es sich bei dem Gehäuse der Prothese (100) oder der Orthese (400) um ein einstückiges Teil handelt.

2. Prothesengehäuse nach Anspruch 1, bei dem das Prothesengehäuse (100) ein Sprunggelenkprothesengehäuse ist und des Weiteren einen Fußbefestigungsabschnitt (132) umfasst, der konfiguriert ist, um das Gehäuse an einem Fußteil (230) anzubringen.

3. Prothesengehäuse nach Anspruch 1 oder 2 oder Orthesengehäuse nach Anspruch 1, des Weiteren umfassend eine oder mehrere Öffnung(en) (191, 192, 193), die konfiguriert ist bzw. sind, um darin ein oder mehrere entsprechende(s) Ventil(e) zur Steuerung des Fluidstroms durch einen oder mehrere der Kanäle hindurch aufzunehmen.

4. Prothesen- oder Orthesengehäuse nach Anspruch 3, des Weiteren umfassend ein oder mehrere Ventil(e) (552, 553) mit einstellbarer Blende.

5. Prothesen- oder Orthesengehäuse nach Anspruch 3 oder 4, des Weiteren umfassend ein oder mehrere Rückschlagventil(e) (554, 555, 556).

6. Prothesen- oder Orthesengehäuse nach einem der vorhergehenden Ansprüche, des Weiteren umfassend eine Öffnung, die konfiguriert ist, um einen Schalter (246) aufzunehmen, der mit einem oder mehreren der Kanäle (501) derart fluidisch zu verbinden ist, dass mit dem Schalter das Prothesengehäuse zwischen einer ersten und einer zweiten Betriebsart umgeschaltet wird.

7. Prothesen- oder Orthesengehäuse nach Anspruch 6, des Weiteren umfassend ein Solenoid (246) als Schalter in der Öffnung.

8. Prothesen- oder Orthesengehäuse nach einem der vorhergehenden Ansprüche, bei dem der Pumpenabschnitt konfiguriert ist, um zwei Zahnräder einer Zahnradpumpe aufzunehmen, und des Weiteren Mittel zur Anbringung der restlichen Pumpe am Gehäuse umfasst.

9. Prothesen- oder Orthesengehäuse nach einem der vorhergehenden Ansprüche, bei dem eine Vielzahl der Kanäle (151, 152, 153, 154, 451, 452, 453) keine Krümmungen mit einem Krümmungsradius von weniger als einem Drittel der Kanalbreite oder des Kanaldurchmessers und vorzugsweise weniger als einem Viertel der Kanalbreite oder des Kanaldurchmessers aufweist.

10. Prothesen- oder Orthesengehäuse nach einem der vorhergehenden Ansprüche, bei dem das Gehäuse aus einem Material mit einer Mikrostruktur besteht, die darauf hindeutet, dass es mittels additiver Fertigung hergestellt wurde.

11. Prothese oder Orthese, folgendes umfassend:
ein Gehäuse nach einem der vorhergehenden Ansprüche;
einen Kolben (221) und eine Kolbenstange (220), die in dem Zylinder des Gehäuses untergebracht sind; und
eine am Gehäuse angebrachte Pumpe (244).

12. Prothese oder Orthese nach Anspruch 11, wenn abhängig von Anspruch 3, des Weiteren umfassend ein oder mehrere Ventil(e) (552, 553) mit einstellbarer Blende.

13. Prothese oder Orthese nach Anspruch 11 oder 12, wenn weiter abhängig von Anspruch 4, des Weiteren umfassend ein oder mehrere Rückschlagventil(e) (554, 555, 556).

14. Prothese oder Orthese nach Anspruch 11, 12 oder 13, wenn weiter abhängig von Anspruch 7, des Weiteren umfassend ein Solenoid (246).

15. Prothese nach einem der Ansprüche 11 bis 14, wenn weiter abhängig von Anspruch 2, des Weiteren umfassend ein Fußteil (230).

## Revendications

1. Boîtier de prothèse (100) ou d'orthèse (400), comprenant :
un cylindre configuré pour recevoir un piston (221) d'un ensemble piston et cylindre (120, 220) ;
une section pompe configurée pour recevoir une partie d'une pompe (244) ; et
une pluralité de passages (151, 152, 153, 154, 451, 452, 453) raccordant le cylindre à la section pompe, **caractérisé en ce qu'**une pluralité des passages (151, 152, 153, 154, 451, 452, 453) sont dépourvus de coude ayant un rayon de courbure inférieur à la moitié de la largeur ou du diamètre du passage, et
dans lequel le boîtier de prothèse (100) ou d'orthèse (400) est une seule pièce.

2. Boîtier de prothèse selon la revendication 1, dans lequel le boîtier de prothèse (100) est un boîtier de prothèse de cheville et comprend en outre une section de fixation de pied (132) configurée pour fixer le boîtier à un composant pied (230).

3. Boîtier de prothèse selon la revendication 1 ou 2 ou boîtier d'orthèse selon la revendication 1, comprenant en outre une ou plusieurs ouvertures (191, 192, 193) configurées pour recevoir une ou plusieurs soupapes respectives pour réguler l'écoulement de fluide à travers un ou plusieurs des passages.

4. Boîtier de prothèse ou d'orthèse selon la revendication 3, comprenant en outre une ou plusieurs soupapes à orifice ajustable (552, 553).

5. Boîtier de prothèse ou d'orthèse selon la revendication 3 ou 4, comprenant en outre une ou plusieurs soupapes antiretour (554, 555, 556).

6. Boîtier de prothèse ou d'orthèse selon l'une quelconque des revendications précédentes, comprenant en outre une ouverture configurée pour recevoir un commutateur (246) à raccorder fluidiquement à un ou plusieurs des passages (501) de telle sorte que le commutateur permette la commutation du boîtier de prothèse entre des premier et second modes de fonctionnement.

7. Boîtier de prothèse ou d'orthèse selon la revendication 6, comprenant en outre un solénoïde (246) en tant que commutateur dans l'ouverture.

8. Boîtier de prothèse ou d'orthèse selon l'une quelconque des revendications précédentes, dans lequel la section pompe est configurée pour recevoir deux engrenages d'une pompe à engrenage et comprend en outre un moyen permettant de monter le reste de la pompe sur le boîtier.

9. Boîtier de prothèse ou d'orthèse selon l'une quelconque des revendications précédentes, dans lequel une pluralité des passages (151, 152, 153, 154, 451, 452, 453) sont dépourvus de tout coude ayant un rayon de courbure inférieur à un tiers de la largeur ou du diamètre du passage et de préférence inférieur à un quart de la largeur ou du diamètre du passage.

10. Boîtier de prothèse ou d'orthèse selon l'une quelconque des revendications précédentes, dans lequel le boîtier se compose d'un matériau ayant une microstructure indiquant qu'il a été fabriqué par fabrication additive.

11. Prothèse ou orthèse comprenant :
un boîtier selon l'une quelconque des revendications précédentes ;
un piston (221) et une tige de piston (220) reçus à l'intérieur du cylindre du boîtier ; et
une pompe (244) montée sur le boîtier.

12. Prothèse ou orthèse selon la revendication 11 lorsqu'elle dépend de la revendication 3, comprenant en outre une ou plusieurs soupapes à orifices réglables (552, 553) .

13. Prothèse ou orthèse selon la revendication 11 ou 12 lorsqu'elles dépendent en outre de la revendication 4, comprenant de plus une ou plusieurs soupapes antiretour (554, 555, 556).

14. Prothèse ou orthèse selon la revendication 11, 12 ou 13 lorsqu'elles dépendent en outre de la revendication 7, comprenant de plus un solénoïde (246).

15. Prothèse selon l'une quelconque des revendications 11 à 14 lorsqu'elles dépendent en outre de la revendication 2, comprenant de plus un composant pied (230).
